# EUROPEAN PATENT SPECIFICATION

(11) **EP 1 105 029 B1**
(45) Date of publication and mention of the grant of the patent: **14.03.2007**
(21) Application number: 99937657.7
(22) Date of filing: 29.07.1999
(51) Int. Cl.: A47K 10/24, B32B 3/06, B32B 3/30, B65H 5/28, A44B 18/00, B65H 35/00

(54) **DISPENSING FASTENER STRAPS**
SPENDER FÜR BEFESTIGUNGSBÄNDER
DISTRIBUTEUR DE BANDES DE FIXATION

(30) Priority: 14.08.1998 US 133991
(43) Date of publication of application: 13.06.2001
(60) Divisional application: 06027081.6
(73) Proprietor: VELCRO INDUSTRIES B.V., Curacao (AN)
(72) Inventor: SHEPARD, William, H., Amherst, NH 03031 (US)
(74) Representative: Müller-Boré & Partner Patentanwälte
(86) International application number: PCT/US1999/017293
(87) International publication number: WO 2000/008992

(56) References cited:
- EP-A- 0 122 981
- US-A- 4 340 633
- US-A- 4 437 854
- US-A- 5 056 682
- US-A- 5 518 795
- US-A- 5 605 729
- US-A- 5 630 526

## Description

### Background of the Invention

This invention relates to fastener strap dispensers, to methods of making and using such dispensers, and to fastener tape suitable for use with such dispensers.

Elongated strips of hook and loop fasteners are used as straps or ties for bundling and wrapping products or for applying identification markings to products.

Previously, a continuous strip of fastener tape has been provided with cross-perforations or lines of weakness spaced at predetermined interval lengths to define individual units which can be detached from one another. In some cases, the tape is wound upon itself to form a supply roll. The user manually uncoils a unit from the roll, finds the line of weakness, and breaks it from the supply roll by tension.

Other items made in continuous sequence as a continuous strip, to be detached at lines of weakness, present similar problems.

EP 0 122 981 A2 discloses a container for a hook and loop fastener comprising two compartments for receiving a respective roll of a hook and loop fastener and only one common outlet in order to dispense the two co-operating tape-shaped components of the hook and loop fastener in a coupled condition.

US 5 630 526 discloses a container for containing a paper roll and dispensing individual parts of the paper roll to be used as a paper towel. The paper roll comprises perforated tear lines along which an individual part of the paper can be separated from the paper roll when a certain pulling force is applied to the end of the paper roll. Further, the container comprises a member which is biased against the paper roll to resist rotation thereof so as to assure application of said certain pulling force.

US 5 518 795 discloses a laminated hook fastener created on a roll and having hook forming cavities in its surface and a second strip carried by a backing roll. The second strip is firmly bonded to the fastener strip on that side opposite the formed hooks.

It is the object of the invention to provide an improved dispenser for dispensing two-sided fastener tape, as well as a corresponding tape and method.

This object is fulfilled by a fastener strap dispenser having the features disclosed in claim 1, a fastener tape having the features disclosed in claim 14 and a method having the features disclosed in claim 25. Preferred embodiments are defined in the dependent claims.

According to one aspect of the invention, the dispenser has a housing defining a receptacle and an outlet. A length of fastener tape is disposed within the housing and is arranged to be dispensed through the outlet. The tape has a strip-form base with two broad, opposite surfaces, and an array of fastener elements extending from one of the surfaces. The base defines tear regions of reduced tear strength formed at spaced intervals along its length, thus defining individual fastener straps between the tear regions. Each fastener strap defines an associated, engageable formation and the dispenser has a detent constructed and positioned to engage the formation of a trailing fastener strap as a leading fastener strap is pulled through the outlet. The detent acts to resist movement of the trailing strap so that the leading strap parts from the trailing strap at a tear region when a sufficient parting tension is applied to the leading strap.

In some embodiments, the fastener strap dispenser has a retarder arranged to provide drag against motion of the fastener tape as the leading strap is withdrawn through the outlet. In a particular construction of such an embodiment, the retarder is a resilient member, such as a leaf spring or a spring-loaded roller, that bears against the fastener tape material as the leading strap is withdrawn.

In some particular instances, the detent is a fixed protrusion over which the fastener tape slides and the formations comprise slits defined by the fastener tape. The protrusion is arranged to engage the slit of a trailing strap as a leading strap is pulled from the outlet. In some such instances the resilient member is positioned to bias the fastener tape toward the fixed protrusion as the leading strap is withdrawn from the outlet, so as to aid engagement of the formation of the trailing strap by the fixed protrusion. In some other instances the resilient member is positioned to bias the fastener tape away from the fixed protrusion as the leading strap is withdrawn from the outlet, so as to aid disengagement of the formation of the trailing strap from the fixed protrusion. In some instances the dispenser has a guide channel defined by the housing to position the formations for engagement with the fixed protrusion as the tape is dispensed.

For applications requiring such a feature, in some embodiments the fastener strap dispenser housing has a mounting boss for permanently mounting the dispenser to a supporting surface.

In some cases the fastener straps used in the dispenser have formations located at the tear regions, while in some other cases the formations of the fastener straps are longitudinally spaced from the tear regions.

In some instances the fastener strap dispenser is proportioned to be hand-held by a user. Preferably, such hand-held dispensers have a total weight of less than about two pounds (0.9 kg), more preferably, less than about 1 pound (0.5 kg). For easy manual manipulation it is preferred that hand-held dispensers have a maximum linear dimension of about 5 inches (127 mm) or less.

In presently preferred embodiments the fastener strap dispenser is used with fastener tape having fastener elements, such as hooks or hook engageable fibers for hook and loop fastening.

In some cases the fastener tape has fastener elements constructed for self-engaging fastening.

In some particularly advantageous constructions, the fastener tape has loops on one broad surface and loop-engageable fastener elements on an opposite broad surface, or has fastener elements on one broad surface and pressure-sensitive adhesive on an opposite broad surface. In some such adhesive-bearing embodiments, the fastener tape also has a release layer extending over the pressure-sensitive adhesive.

Sometimes the fastener tape used in the dispenser is wound in roll form while sometimes the fastener tape is in Z-fold form.

In certain of the hand-held embodiments, the dispenser housing has a curved outer surface to receive an outer surface of an article to be wrapped.

The dispenser has, for some applications, a retarder positioned to resist dispensing of the tape at the outlet. The retarder is manipulable by a user to provide increased resistance to a trailing fastener strap to facilitate separation of a leading fastener strap from the trailing strap at an associated tear region when a sufficient parting tension is applied to the leading strap.

In some cases the dispenser has a severing device at the outlet of the housing. The severing device has a sharp edge for cutting through the base of the tape when forced against one of the broad surfaces of the tape. The severing device may be, for example, an exposed edge of a retarder.

In another aspect of the invention, a length of fastener tape has an elongated strip-form base of uniform width, with two broad, opposite surfaces and tear regions of reduced tear strength formed at spaced intervals along its length. Individual fastener straps are defined between consecutive tear regions and the strip-form base defines engageable formations for engaging the strip-form base to tear the fastener tape along an associated tear region. The fastener tape also has an array of fastener elements extending from at least one of the broad surfaces of the base.

In some instances the formations comprise slits extending through the base. In one particular embodiment, discussed below, the slits are u-shaped and the base and array of fastening elements are a single, unitarily molded structure.

In some instances, the fastening elements of one of the broad surfaces are adapted to engage fastening elements of the other broad surface. For example, the fastening elements of one broad surface may comprise loops and the fastening elements of the other broad surface may comprise loop-engaging fastener elements.

In another aspect of the invention, a method of using a dispenser to wrap an article is disclosed.

The dispenser of the present invention provides an efficient mechanism for singulating fastener straps from a supply. Consistent and predictable dispensing of fastener straps, requiring minimal effort on the part of the user to find separation lines of adjacent straps, as provided by the present dispenser, can aid a user in wrapping articles quickly and accurately.

In particular, hand-held embodiments can be useful for dispensing tape as the tape is being wrapped around an article.

### Brief Description of the Drawings

FIG. 1 is a side view of a dispenser holding a roll of fastener strap material, its cover removed;
FIG. 1A is a front view of the dispenser;
FIG. 1B is a side view of the dispenser similar to FIG. 1, with the cover shown in its position for loading;
FIG. 1C is a side view of the claw included in the dispenser in FIG. 1;
FIG. 1D is a frontal view of the claw shown in FIG. 1C;
FIG. 2 is a diagram of a tensioning system of the dispenser of FIG. 1;
FIG. 2A is a diagram of the tensioning system illustrated in FIG. 2, with an alternatively shaped spring end;
FIG. 2B is a diagram of the tensioning system illustrated in FIG. 2, having a spring with a thinned cross section;
FIG. 3 illustrates a fastener strap being pulled from the dispenser;
FIG. 4, similar to FIG. 3, shows the strap material being engaged by a detent immediately before separation of the leading strap from the roll;
FIG. 5 is a top view of the strap of FIG. 4 being engaged by the detent;
FIG. 6 is a view similar to FIG. 1 of another dispenser construction, with an angled dispenser section, while FIG. 6A is a side view of the cover used with the dispenser of FIG. 6;
FIG. 6B is a side view of a dispenser, having three spring rollers to apply pressure to the strap material to be dispensed;
FIG. 6C is a view similar to FIG. 1 of a dispenser construction, having the tensioning system illustrated in FIG. 2B;
FIG. 7 is a top view of two straps defined by a line of perforations and having a longitudinal slit in adjacent ends;
FIG. 7A is a magnified view of the perforation and slit of FIG. 7;
FIG. 8 is a top view of two adjacent straps, having another type of formation at their juncture;
FIG. 8A is a magnified view of the features illustrated in FIG. 8;
FIG. 8B is a top view of two adjacent straps, having another type of formation at their juncture, with the oval cut formation somewhat displaced from the juncture;
FIG. 8C is a magnified view of the features illustrated in FIG. 8B;
FIG. 9 is a top view of two adjacent straps, having another type of formation at their juncture;
FIG. 9A is a magnified view of the features illustrated in FIG. 9;
FIG. 10 is a longitudinal cross section view of a length of strap material having fastener hooks on one side and loops on the other side;
FIG. 10A is a magnified view of the material of FIG. 10;
FIG. 11 is a longitudinal cross-sectional view of a means for manufacturing an integrally molded engaging member with backing material attached;
FIG. 12 is a variation from FIG. 11, based on the same general principles;
FIG. 13 is a side view of a portable fastener strap dispenser;
FIG. 14 is an enlarged cross-sectional view taken along line 14-14 of FIG. 13;
FIG. 15 shows a side view of the portable dispenser of FIG. 13 being used to wrap fastener strap about a cable bundle;
FIG. 16 shows a front view of the portable dispenser of FIG. 13 being used to wrap fastener strap about a cable bundle;
FIG. 17 is an enlarged side view of the portable dispenser of FIG. 13 with a near side of the front guide channel removed;
FIG. 18 is an end view, as seen from direction 17-17 in Fig. 13, with the strap removed;
FIG. 19 shows a portion of a roll of fastener strap for use with a modified dispenser of FIG. 13;
FIG. 20 shows a portion of a roll of fastener strap for use with a modified dispenser of FIG. 13;
FIG. 21 is a side view of a portable fastener strap dispenser similar to the dispenser of FIG. 13;
FIG. 22 is a side view of a replacement roll of fastener strap for use in the dispenser of FIG. 13 or FIG. 21.

### Description of Embodiments

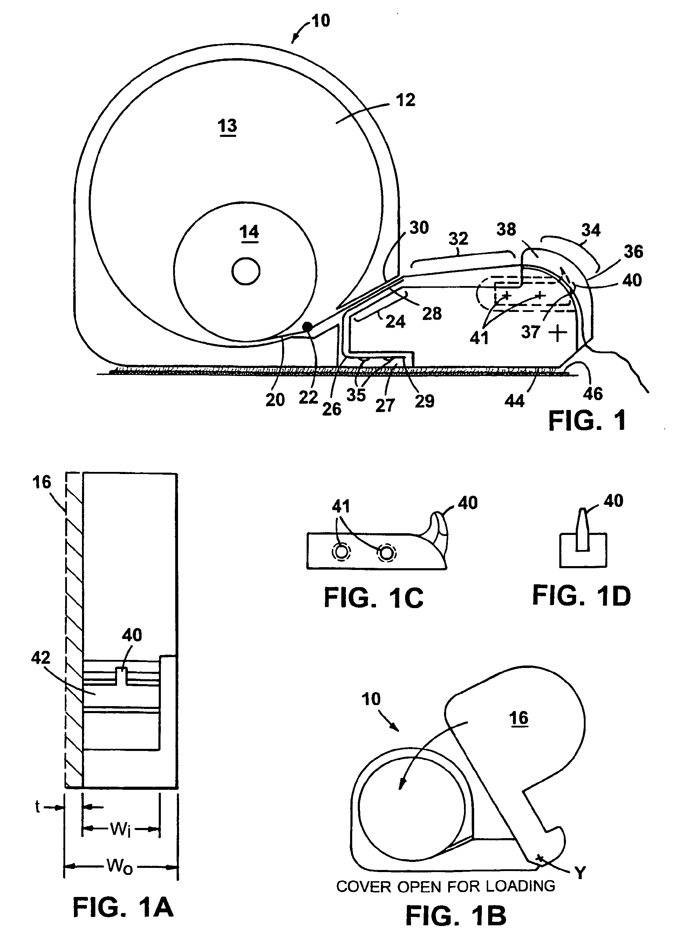

Referring to FIGS. 1-6, dispenser **10** has a housing structure that defines supply receptacle **12,** which is shaped to loosely hold roll **14** of fastener strap (or other strip form member), to permit rotation of the roll **14.**

The receptacle **12** has a back wall **13,** and is open at its front to enable loading by axial insertion motion of the roll **14.** In FIG. 1B, tape cover **16** of the dispenser, shown in open position, is pivotally mounted to rotate about axis Y to closed position where it locks in place. After the roll **14** is loaded into the dispenser below, cover **16** pivots closed about axis Y, to confine the roll **14.**

In one example, receptacle **12** and cover **16** are configured to receive a roll **14** of up to 7.5 inches diameter while in other embodiments the dispenser may be larger or smaller based on user needs. The number of straps in the roll **14** is of course determined by the roll diameter, the wind tension, and the length and thickness of the individual straps. In one embodiment, the dispenser holds a roll of 75 straps, each 18" long by 0.5" wide, the straps having thickness of about .040 inches, forming a roll of about 7.0 inches diameter. The wind tension is sufficient to produce a firm roll which does not easily collapse and cause jamming at the separation pin **22.**

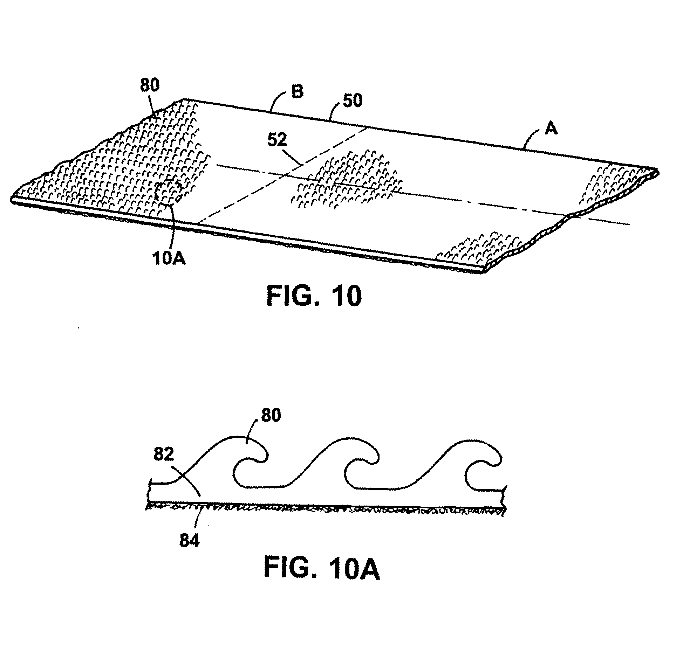

The strap material in certain cases is a fastener hook tape or fastener loop tape, each adapted for hook and loop fastening. In the presently preferred embodiment, it is a combination tape having fastener hooks on one side and loops on the other as shown in FIGS. 10 and 10A. As will be described further below, in many applications the hook comprises a plastic strip having an array of fastener hooks molded integrally with the base layer and the loop component is a light-weight, needled, and stretched product that is in situ bonded to the base of the hook strip by the adhesive properties of the synthetic resin layer that forms the corresponding side of the base layer. The present embodiment of FIGS. 1-6 is constructed to dispense such two-sided fastener tape.

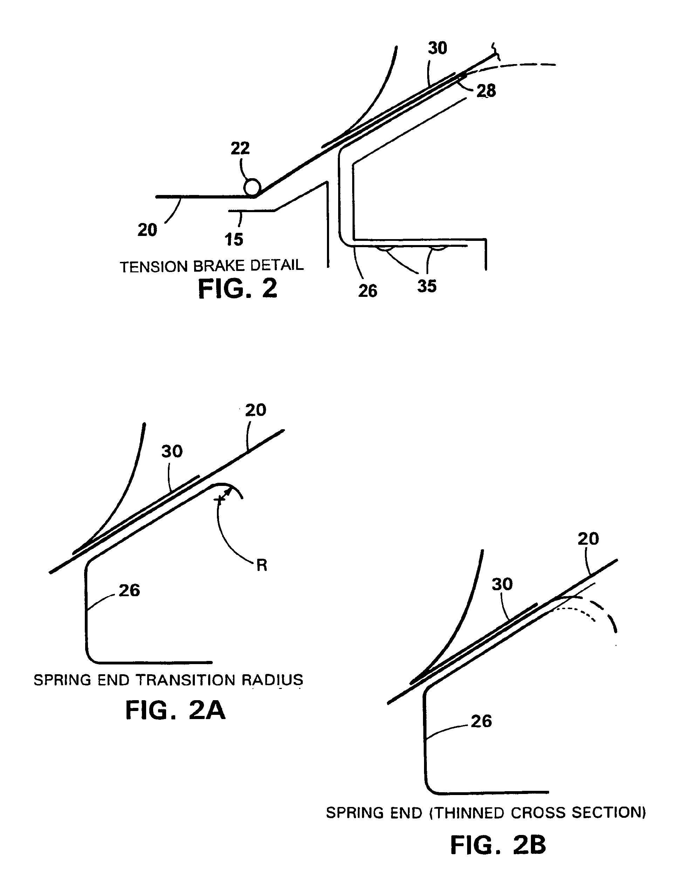

As shown in FIG. 1 and in detail in FIG. 2, in passing from the supply roll **14** along a removal path, the strap **20** being removed first passes over separation pin **22.** Pin **22** extends parallel to axis Z of the supply roll **14,** and is located at a height even with support surface **15.** The effect of the strap passing under the separation pin, combined with the weight of the roll that resists its vertical movement, serves to break the bond between adjacent hook and loop sides of the strap that is rolled upon itself. Following separation pin **22,** the tape **20** passes through a retarding path **24** defined between a flat spring **26** and a land surface **30** defined by the housing structure of the dispenser.

In the embodiment shown, spring **26** is of blue spring steel, flat in orientation. It extends from mount **29** as a cantilever and has an end portion **28** that extends substantially parallel to land **30,** against which it is spring biased, such that, in the absence of tape **20,** the spring portion **28** engages the land **30.** Two screws **35** are perpendicular to the plane of the spring, and go up vertically. Parallel to the right hand side beyond the screw holes **35** is the end of the pocket **27.** The depth of the pocket **27** provides enough depth to recess the screw head.

In the presence of the tape, respective faces of the fastener tape are engaged, the spring serving to maintain the emerging part of the strap raised to be grasped and to frictionally retard the tape as the tape is pulled from the dispenser. The moderate predetermined drag provided by the retarding path ensures that the product does not pull out too freely during normal application of withdrawing tension, and it provides resistive tension for enabling the initial phase of the singulating action now to be described.

Following the retarding path, the fastener tape extends freely through access region 32 at which it can be initially grasped by the user. The tape path then extends through guide channel **34** which is open at the top. Guide channel **34** is defined by two side guide members **36, 37,** disposed to be engaged by the edges of the tape, and a downwardly curved lower surface **38** against which the user draws the tape, as the user pulls downwardly and rightward against the resistance provided by the spring which deflects. In this embodiment, one of the guide members **37** is a portion of the structure of the removable cover **16,** and the other side guide **36** is integral with the main housing of the dispenser.

Each guide **36, 37** is sufficiently rigid to engage and guide the edges of the strip material which it is adapted to dispense. The two guides **36, 37** thus provide a centering action, to guide the tape so that it registers with a detent device **40** located in the guide channel **34.**

In the present embodiment, the detent device **40** is a stationary claw, shaped much like a cat's claw, directed upwardly and hooked backwardly to oppose motion of the strip. The claw tapers slightly to a dull point, which is disposed to be slidably engaged by the underside of the tape as the user, with downward and forward force, draws the tape through the guide channel, against curved surface **38.** The tape thus slides over the apex of claw **40.** In this embodiment the claw **40** is centered in the middle of the guide channel between the two side plates **36, 37.** The shape of the claw is shown in FIGS. 1C and 1D.

In FIG. 1A, a side view of the dispenser (cover **16** shown by dashed lines), the elevation of claw **40** from the body **42** of the claw holder is illustrated. In this embodiment claw **40** is formed integrally with the structural body **42,** the claw profile having been provided by a separate piece that is inserted into the body **42.** The claw body has mounting holes **41** for attachment.

(In production versions formed by injection molding, the claw is of impact structural resin, e.g. acrylonitrile butadiene styrene (ABS)/or high impact polystyrene, integrally formed with the dispenser housing.)

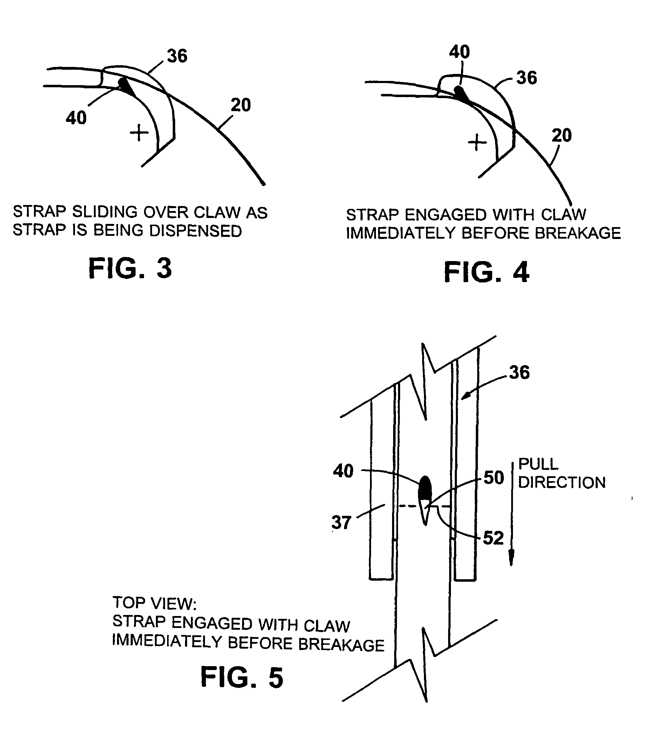

The detent **40** between the two side plates 36 and **37,** rounded as to accommodate sliding movement of the tape **20,** stands up and indents the tape as the tape, being drawn by the user from the dispenser, is pulled in rightward, downward motion (FIG. 3), overcoming drag applied to the tape by spring **26.** When a formation provided in the strip material at the juncture of the leading strip units and the following units reaches the claw, the claw penetrates the substance of the tape, as shown in FIG. 4 and resists further motion of the following strip unit to the degree that the line of weakness ruptures under user's tension and the leading strip unit is separated from the supply.

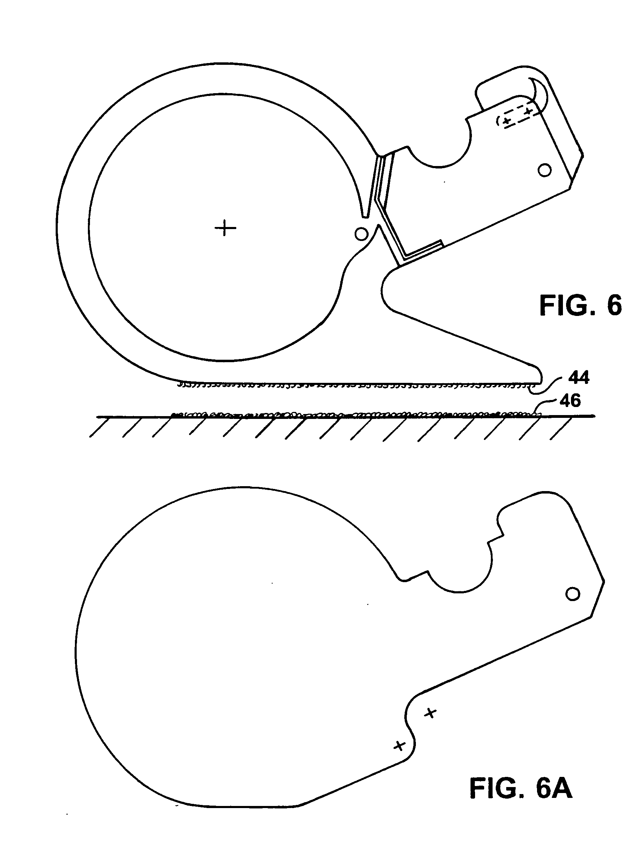

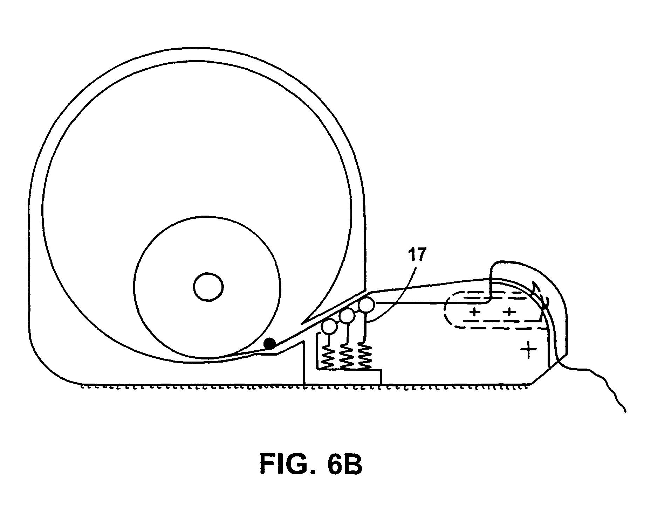

As illustrated in FIG. 6B, in order to work with the fastener having tape adhesive on its backside, three spring rollers **17** replace the flat spring **26** and engage the adhesive. The rollers are adjustably spring loaded; drag is provided to the strap by the contact of the non-adhesive side of the product against the body of the dispenser.

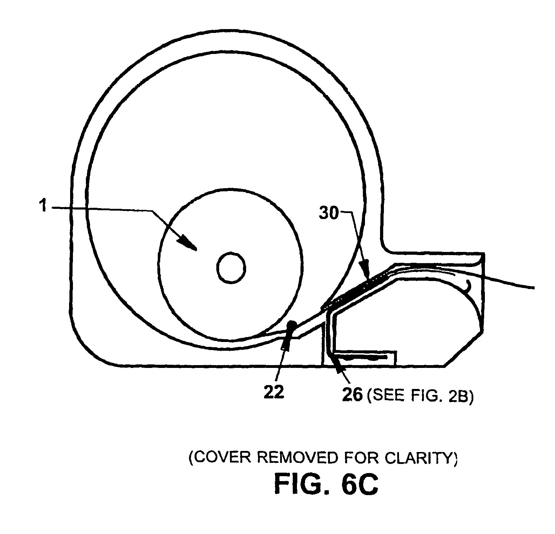

FIGS. 6 and 6A illustrate an alternative configuration of the dispenser, where the dispensing section is angled to enable the user to pull strip material straight out of the dispenser at an upwardly inclined angle, when the dispenser is resting on a flat surface.

In another embodiment, shown in FIG. 2A, spring **26** has a curved end. This shape enables the user to smoothly pull a strap out of the dispenser without scratching the strap's surface, and without damaging printing which might be placed on the strap. Alternatively, the end of the spring **26** can have a thinned cross section, as illustrated in FIG. 6C and in detail in FIG. 2B. The flexibility provided by the thinned section enables the strap to be pulled out of the dispenser without relieving the drag provided by rearward portions of the spring. When pulling tension is discontinued, the end of the spring restores toward its straight configuration, lifting the fastener into position to be grasped.

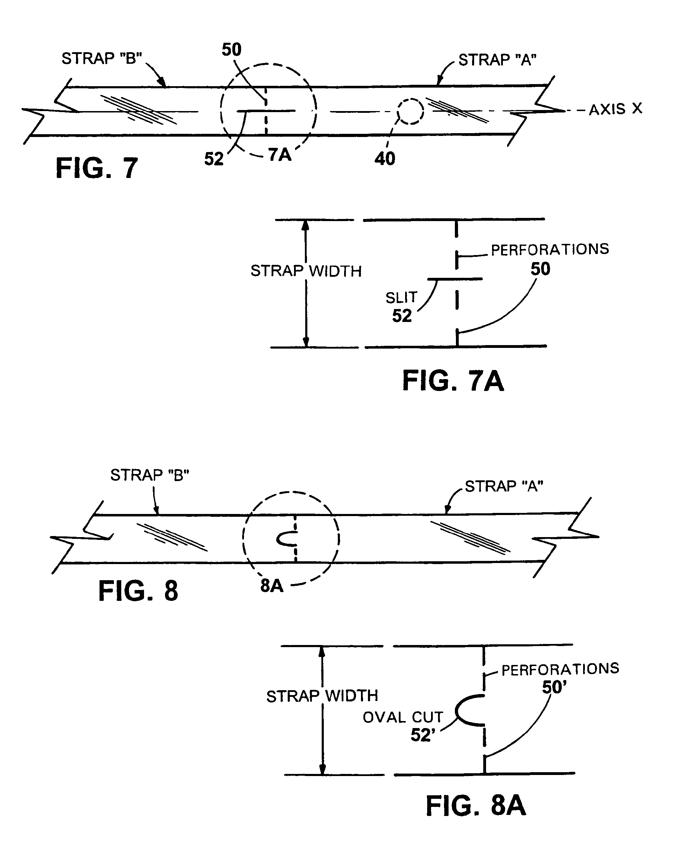

Referring to FIG. 7, leading and trailing straps A and B are shown with a line of perforations **50** between them, extending from edge to edge of the strap, perpendicular to the longitudinal center axis X of the strap.

The magnified view of FIG. 7A shows a blown-up version of the slit **52** and perforations **50.** In this embodiment, the slit is approximately 1/2 inch long, 1/4 inch in each of straps A and B, and is shown centered widthwise and extends perpendicular to the line of perforations **50.** The slit and perforations are readily formed with a rotary die system or a stamping system or a laser cutting system through which the strap material is passed before it is formed in rolls.

A longitudinal slit **52** cut through the thickness of the strap is formed in the adjacent end regions of straps A and B at their juncture, this slit being aligned with longitudinal strap axis X.

As Strap A is pulled from the roll **14,** it moves across the detent **40,** shown in dotted lines. Referring also to FIG. 5, the detent claw is in axial alignment with the center axis X of the strap and slit **52.**

As slit **52** approaches the detent **40,** the claw feature **40** enters the leading part of slit **50** defined in lead strip A. Hook **40** penetrates the thickness of the tape, and, under downward and rightward tension applied by the user to the lead unit, the tape is free to move downwardly against the curved surface **38.** The strap continues to move from the dispenser until the end of the slit **52,** defined by the trailing strap unit B, reaches the claw. The claw resists further movement of the strap and as the lead strap continues to be pulled by the user, the materials breaks at the line of perforations **50,** freeing the lead strap unit A from the supply. The spring **26,** due to its strength and orientation, raises the portion of the trailing strap unit emerging into region **32,** making the unit B available to be grasped. The action of spring **26** pulls strap B from the claw **40.**

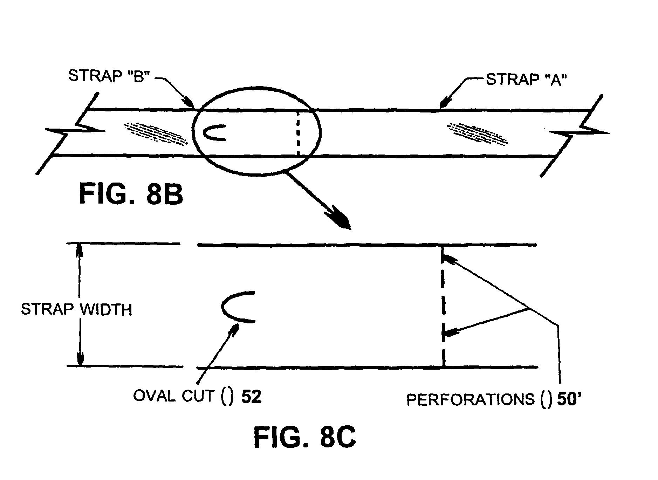

FIG. 8B illustrates still another tape construction which has a line of perforations **50'** similar to those in FIGS. 7 and 8. However, the oval formation **52',** which in FIG. 8 is placed directly at the line of perforations, is moved some distance into the strap. This arrangement results in the trailing strap protruding some distance from the dispenser after the leading strap has been severed, making it even more convenient for the user to grasp the next strap.

The length and spring force of the spring **26** is selected to perform its dual function of applying drag as the strap is withdrawn, and of raising the strap material when the preceding strap breaks at the perforations, to position the next strap in a convenient manner for the user to grasp.

Another tape construction, shown in FIG. 8, has a line at perforations **50'** similar to those of FIG. 7. However instead of a straight slit, there is an oval cut formation **52'.** The oval cut formation provides a benefit in that it helps avoid longitudinal rips in those tape constructions which are susceptible to longitudinal ripping failure. The oval formation **52'** serves to spread the load applied by claw **40** to the trailing strap, and thus lessen the stress applied to the trailing strap. The oval formation is advantageous, for instance where the base layer of fastener hook tape being dispensed is thin and not sufficiently reinforced to prevent propagation of a failure line. A larger oval formation helps keep the tape from catching on the claw.

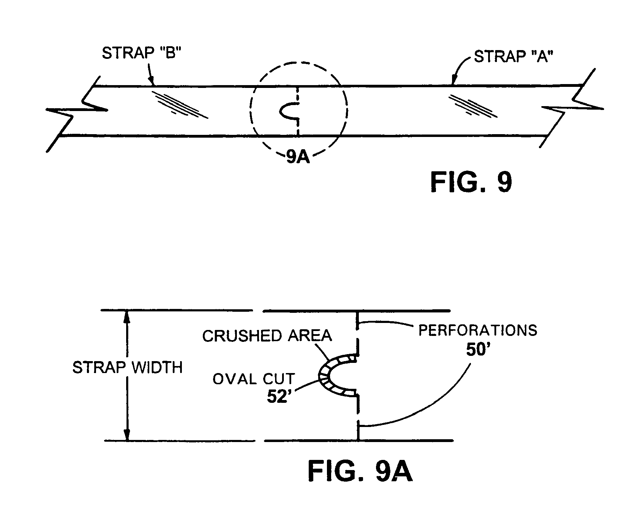

The strap of FIGS. 9 and 9A shows straps, perforations, and cut through similar to those of FIG. 8, with an additional crushed area **54** around the oval, formed by a die that deforms by compression. The crushed or depressed area enables the user to see the beginning and end of the strap when it is in non-broken form, as it approaches the detent.

For forming the crushed area in a hook or loop fastener tape, a land is provided in the die or stamping tool of about 1/16" width; its pressure crushes all the features of the hook or the loop and is shaped to cut through on an edge of the crushed region to form the oval cut.

The braking action of the spring which enables tension to be applied on the strip and which prevents overfeed, enables the mechanism to be used in one-handed operation. For this purpose, the device is attached to a table, or is otherwise secured to resist the tension applied by the user to the dispenser. (In the preferred embodiment shown, hooks **44** are integrally molded in the base of the dispenser, and the dispenser is packaged with a strip of adhesively backed loop material **46.** The loop material is adhered to a bench or other surface, and the dispenser is releasably secured to the loop materials, by resting upon it, with its hooks engaged in the loops.)

The side guides **36, 37** provide alignment between the claw feature **40** and the slit feature **52, 52'** that have been cut into the straps. The bigger the hole that is slit or punched through the strap, the lower are the alignment requirements. However, a degree of strength is required so that the straps stay connected until it is necessary that they be broken. As one increases the size of the feature which the claw **40** engages to hold the strap back, one decreases the available strap width that remains for strength purposes.

In another alternative, an angled slit is provided that extends along the length of e.g. the center half of the tape, so there is a 1/4" on each side, but angled. In this embodiment, the claw feature **40** is able to find it, regardless of the alignment. However, as the height of the claw feature of the detent **40** increases, the stability of the narrow web riding on the top of it without guides becomes less sure. The detent **40** has to be sufficiently high to find the slit; in the case of a diagonal cut, it must extend through sufficiently that the strap cannot ride off it so that it is captured.

The slit or other formation in the strap does not have to be symmetrically disposed or centered so long as the dispenser enables the strap to be guided into engagement with the hook or hooks or other detent.

In another embodiment, the dispenser is manufactured by injection molding techniques as two halves constructed to be releasably joined (or permanently joined with a roll of tape inside with which the dispenser is provided as a disposable unit). One injection molded half, for instance, is constructed to slide over the other half to capture the supply roll.

In a preferred example of such case, the separation pin **22,** the land **30,** and the claw feature **40** are all injection molded as integral parts of the plastic housing structure.

The spring **26** may be separately formed and inserted into the assembled structure, or it may be injection molded to become integrated with one of the injection molded parts. The spring may for instance be blue spring steel or an impact-resistance plastic.

Referring to FIGS. 10 and 10A, straps A and B are comprised of rows of molded hooks **80,** alternate rows facing in opposite directions. The hooks molded integrally with base layer **82** and the straps have **in situ** bonded to their lower surface non-woven loop material **84** that forms loops of suitable dimensions to be releasably engaged with hook **80.** In a preferred form for use in low cost applications, the non-woven fabric is a needled and stretched loop material described further below.

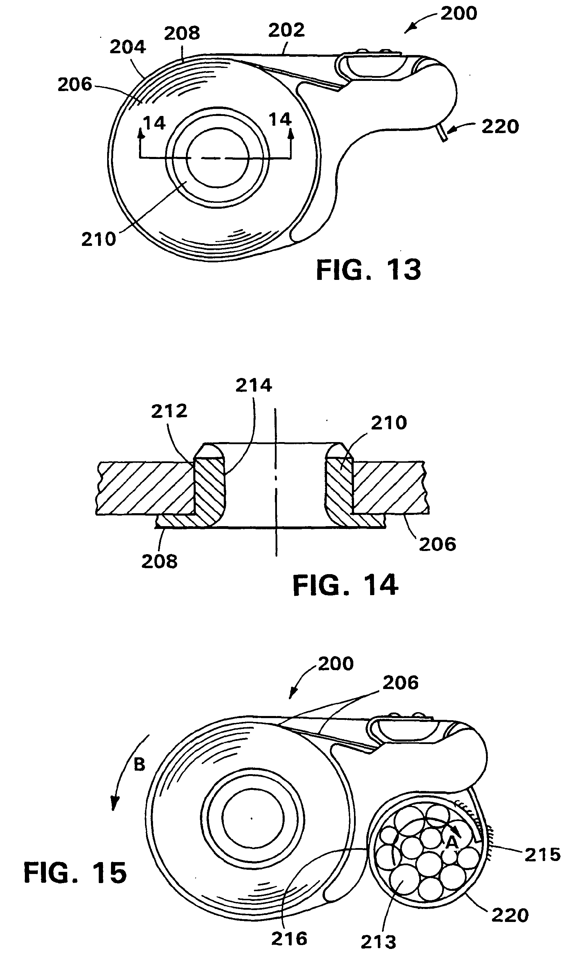

As illustrated in FIGS. 13-18, hand-held portable dispenser **200** has a housing **202** with a supply receptacle **204** for holding a roll of fastener strap **206.** The receptacle **204** has a back wall **208** and is open at its front to enable coaxial loading of the roll over a mounting hub **210.**

The roll **206** is free to rotate upon the mounting hub which, as shown in FIG. 14, has a flanged outer edge **212** to help retain the roll on the hub. A user's finger may be inserted through an opening **214** through the mounting hub to aid in winding dispensed fastener strapping **220** around an article to be wrapped.

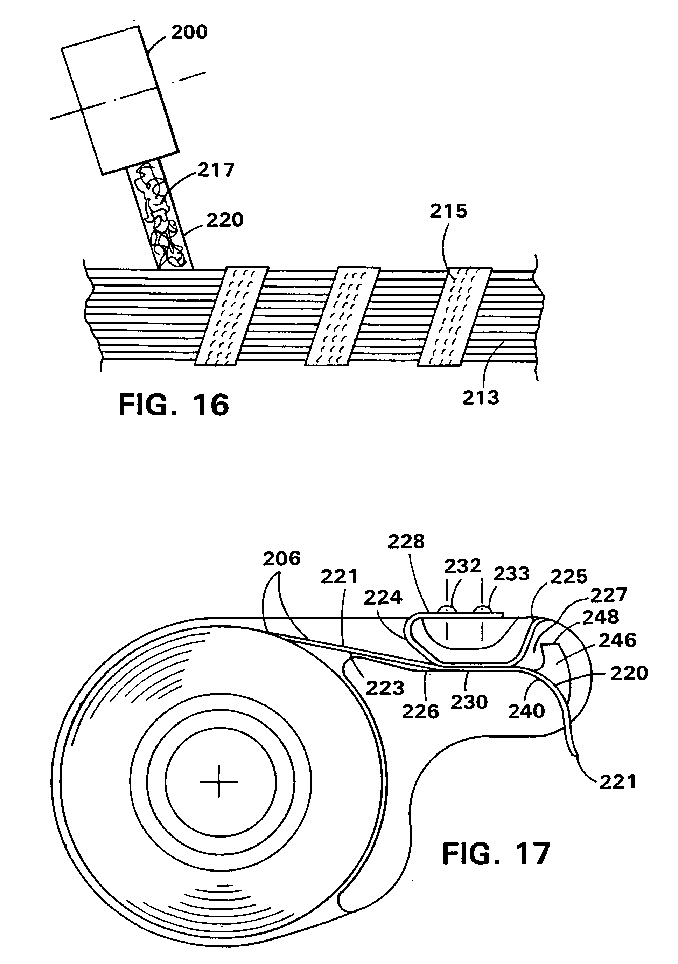

In use (as shown in FIG. 15 and 16), dispenser **200** is held in one hand and brought to an object to be wrapped (such as the cable bundle **213** shown). The user first pulls the strapping being dispensed **220** up to clear dispenser claw **246** (FIG. 17, discussed below), pulls enough strap out of the dispenser to make one full revolution about the package, and engages the fastener elements **215** of one side of the strap against the loops **217** of the other side of the strap (e.g., at the free end of the strap) to form the first complete "strapping" about the package or bundle. With the once free end of the strap secured to the strap itself, the user may then complete more revolutions of strapping about the package by, for instance, either rolling the bundle (arrow **A)** or spinning the dispenser (arrow **B)** about the bundle to dispense more strap.

Alternatively, a user may pull individual straps from dispenser **200,** separate them from supply roll **206,** and then proceed to wrap the straps about the bundle to be wrapped.

As illustrated in FIG. 15, housing **202** has an external surface **216** shaped to fit against the article **213** to be wrapped, so that a user may easily spin the dispenser **200** around the article **213** as the fastener strap **206** is dispensed. Thus, dispenser **200** is particularly useful for wrapping wire and cable bundles in a helical pattern (FIG. 16).

One of the advantages of using a two-sided fastening strap, as illustrated in FIGS. 15 and 16, is a cushioning effect of the strap against bundle **213** provided by the loop side **217** of the strap. This arrangement helps prevent marring or scratching of sensitive surfaces. Also, wrapped bundle **213** has exposed hook side **215** of dispensed fastening strap **220** for securing bundle **213** against other surfaces (e.g., to an inner surface of a computer chassis) to keep the bundle in place.

For a given application, such helical bundle wrapping can be performed quickly and efficiently. By pre-determining the diameter of the bundle to be wrapped and the helix length desired, strap roll **206** can be manufactured having individual straps (e.g., strap "A", strap "B" FIGS. 7-9) of sufficient length to accommodate full revolutions at the beginning and end of the helix to engage the strap to itself while also providing the predetermined helix length. Perforations and, optionally, formations, can be provided on individual straps to facilitate tearing off individual straps as discussed above.

As shown in FIG. 17, from supply roll **206** a fastener strap portion being dispensed **220** passes between a flat spring **224** and a land surface **226** of the housing **202** and through dispenser outlet **227** which is defined by the separation of spring **224** and land surface **226** on the side opposite supply roll **206.**

Spring **224** is formed of stainless steel (e.g., 1/4 to 1/2 hard spring steel). The spring extends from a mounting base **228** as a cantilever and has an intermediate portion **230** that extends substantially parallel to land surface **226,** against which it is biased, such that, in the absence of fastener strap **220,** the spring presses against land **226.** Two self-tapping screws **232, 233** secure spring **224** to housing **202.** Also, spring **224** can be secured to housing **202** by molded plastic features (not shown). In an alternate embodiment, not shown, spring **224** is unitarily molded of the same material as housing **202.**

After initial loading of supply roll **206** in dispenser **200,** the free end **225** of spring **224** is lifted to allow positioning of strap **220** between spring **224** and land surface **226** so that free end **221** of strap **220** is graspable from dispenser outlet **227.** When strap **220** is threaded as shown, opposite faces **221, 223** of the fastener strap **220** are engaged, respectively, by spring **224** and land **226.** Thus, strap **220** is frictionally retarded as it is pulled from dispenser **200.** The moderate predetermined strap drag provided by spring **224** ensures correct strap tension during dispensing so that the product **206** does not pull out too freely during normal strap application, and it also helps to engage strap **220** upon the detent claw described below.

The strap dispensing path extends through a guide channel **234,** as shown in FIG. 17, defined between two side members **236, 237,** land surface **226** of housing **202,** and spring **224.** Outboard of spring **224,** surface **226** has a downwardly curved portion **240.** Guide members **236, 237** provide a centering action, to guide strap **220** so that it registers with a rigid claw **246** positioned in the guide channel beyond spring **224.** Claw **246** extends from surface **226** and has a distal edge **248** for engaging corresponding notches **52, 52'** (FIGS. 7 and 8) of strap **220** as the user draws the strap through the guide channel. Spring **224,** while providing strap tension, biases the strap downwardly toward claw **246** to help ensure engagement of the strap and the claw edge. Claw **246** is centered between the guide members, and its shape is shown in FIGS. 16 and 17. The claw **246** is formed integrally with housing **202** in a single molding process.

Separation of a leading strap (e.g., strap "A" FIGS. 7-9) from roll **206** using hand-held dispenser **200** is achieved as in the above described dispenser embodiments, however, in this portable embodiment spring **224** acts downwardly on strap **220.** Thus, a user must pull a leading strap upward with enough force to disengage corresponding formation **52, 52'** (FIGS. 7-9) from claw **246** to initiate strap dispensing.

Therefore, it is especially advantageous in this embodiment to provide strap supply **206** having strap formations **52, 52'** (FIGS. 7-9) some distance from strap perforations **50'** (as shown in FIG. 8B-8C) making it more convenient for the user to grasp the next strap to be dispensed.

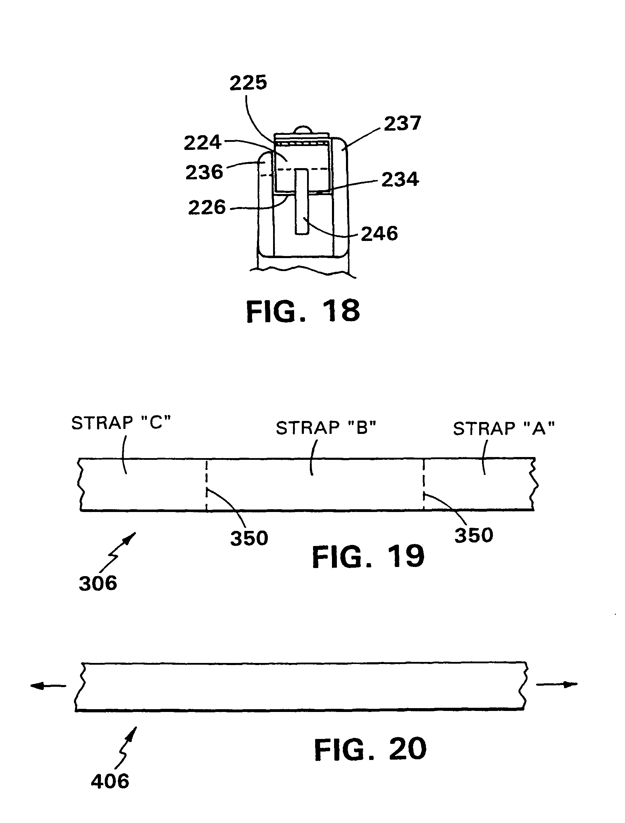

FIG. 18 shows a supply of strap **306** having perforations **350** but without formations for engaging a detent claw. Such a strap construction can be used with a modified dispenser **200** without a detent claw (i.e. - surface **226** being smooth, continuous). For example, a user can press free end **225** of spring **224** or a finger against the portion of strap **306** being dispensed, forcing that portion of strap **306** against surface **226.** In this manner, a user may provide sufficient resistance to a trailing strap (e.g., strap "B" or "C" in FIG. 19) while pulling the free end of a leading strap (e.g., strap "A" in FIG. 19) to separate the leading strap from the trailing strap along the closest exposed perforation line **350.**

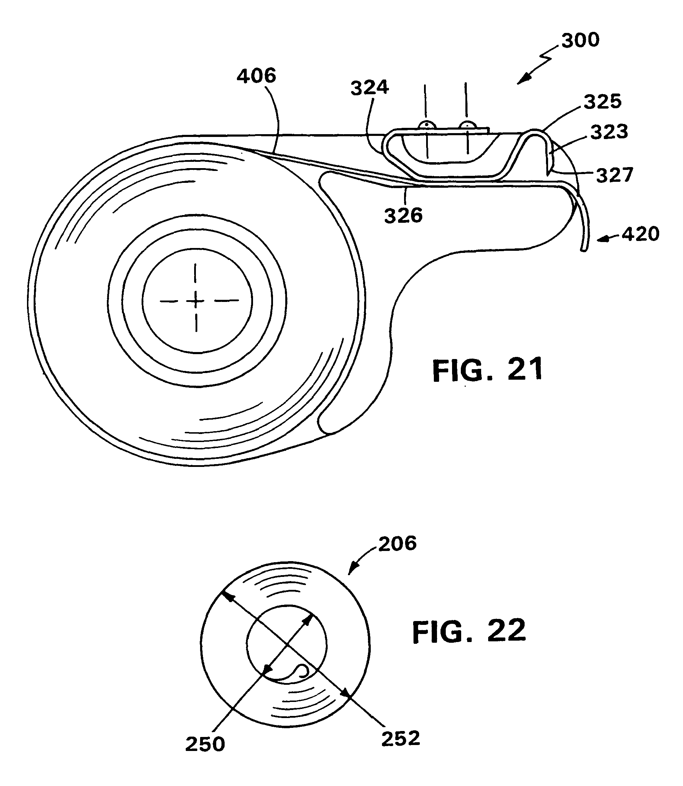

FIG. 20 shows a supply of strap **406** without perforations and without formations for engaging a detent claw. Such a strap construction can be used with the dispenser **300,** shown in FIG. 21, having a blade **323** positioned near the outlet for severing the strap **420** being dispensed. Blade **323** has a severing edge **327** and is formed at a downwardly curved free end **325** of spring **324.** Severing edge **327** faces flat (e.g., without a claw) surface **326** (FIG. 17). After withdrawing a sufficient length of strap **420** to wrap an article, the user can depress free end **325** of spring **324** toward surface **326** with enough force to sever the strapping with edge **327.** Thus, a user can cut a strap of any desired length depending on the particular wrapping application.

A supply roll **206** suitable for use in portable dispenser **200** is illustrated in FIG. 22. Roll **206** has an inner diameter **250** sufficiently large to fit over mounting spool **210** and sufficiently small to be retained by mounting spool flange **212.** Roll **206** also has an outer diameter **252** limited only by the size of receptacle **204.** One example of acceptable roll dimensions is an inner diameter of 1.5 inches and an outer diameter of 3 inches. With typical strap thickness of .040 inches, as discussed above, these dimensions result in a total strap length of approximately 5 yards. The number of strap units (e.g., strap "A", strap "B", etc. in FIGS. 7-9) per roll **206** will depend on the spacing of perforations **50, 50'** (FIGS. 7-9) as discussed above.

The concepts that have been presented have wide application where singulation of a fastener strip product is required. Singulation with one hand is enabled.

Using the hook and loop product from any of the above discussed embodiments for the dispenser offers advantages of speed: once it is wrapped around the product, it sticks to itself and is secure. Identification information can be printed on the hook or loop side of the product.

Another advantageous feature is that the hook and the loop product can be reused. For instance, if wires, fiber optics or other items should need to be added or removed from the bundle, the strap may be undone, the item added or removed, and the strap re-engaged upon itself. The hook and loop product is not significantly affected by moisture, cold, oil, grease and other contaminants and maintains good appearance and its action is still secure after multiple openings and closings. Furthermore, by using the dispenser and applying the tape with the loop side in, a cushioning effect is obtained upon the objects being bundled, preventing abrasion or bruising.

An application is in bundling wires, cables, and hydraulic and pneumatic lines. In the case of telecommunication fiber cables where two-sided hook and loop products are commonly used, the dispenser, for instance, enables significant reduction of the time necessary to singulate straps.

The dispenser may be employed by packers for household moving, to put tape on boxes, for instance with pressure-sensitive materials. In one such case a hook tape is provided that has pressure-sensitive adhesive on its back side and perforated as illustrated here, and another tape which is loop that also has pressure-sensitive adhesive, and is perforated in the same way. Pre-formed pieces of those two fastener components are rolled upon themselves and the rolls mounted in a dual dispenser, side by each, to enable application of each to bags, boxes, etc. to provide a releasable closure system that may be opened and closed repeatedly.

This dispenser may be modified in many ways while still employing important features. In one case a spring lever is advantageously added to the side of the dispenser, associated with an advancing mechanism that is activated by pushing the lever down, to advance the strap. At the time one releases the lever, one would cut a strap of desired length much like the action of traditional paper tape dispensers. This permits use of the dispenser also with strips that do not have the perforated lines of weakness and other features.

The dispenser can also be automated in which the pulling tension is applied by e.g. a robot or another device that grasps the free end and draws it from the dispenser as in the manner described.

The dispenser can be used with a variety of fastener strip material, where the strap material has arrays of fastener elements on both of its sides which are capable of being releasable fastened together. Examples of such elements are hooks and loops. The hooks can be molded integrally with a base layer and the material providing loops including fibers that serve the function provided by textile loops can be joined by the material of the base layer. The loop side of the material can advantageously be a low-cost non-woven web of entangled fibers of substantial tenacity with a basis weight of between 51 g to 71 g per 0,8361 m² (1.8 to 2.5 ounces per square yard).

The loop material can advantageously comprise a batt of loose, staple fibers that have been entangled and form a non-woven fabric of fibers joined at entanglements, with loops of some of the fibers extending from at least one side of the fabric. Advantageously, needling of the batt is employed to form the entanglements. In some embodiments, the non-woven fabric is bonded in a stretched state in which tightened entanglements form knots. The non-woven fabric can also be bonded in a stretched state by resin integral with the material that forms the hook-form fastening elements.

As has previously been indicated with reference to FIGS. 7-10, fastener material suitable for use with the dispenser can be prepared in the form of an extended length of strip material defined as a series of detachable straps. For use with the dispenser detent, the material has formations including weakened parting lines at spaced intervals along its length and at least one formation constructed and arranged to be engaged by the detent to detain the remaining length of fastener material while the leading strip is detached by rupture at the parting line by tension applied to the parting line.

This material is provided with sufficient lateral stiffness to enable it to be guided by edge guides, to register the detent with the formation at the leading end of each strip. Furthermore, it is advantageous that the material have sufficient thickness and stiffness to spring from a bent configuration as it is pulled across the detent to a more planar shape when the leading strip is broken from the remaining material so that the freed end of the remaining material lifts from the detent in a position where it can be readily grasped by a user for dispensing the next successive strip as the new leading strip.

Various types of fastener materials having pressure sensitive adhesive on the side opposite the fastening elements are provided with a release layer, and the lines of perforations are made through both the fastener material and the release layer, thereby enabling simultaneous separation of all layers by the dispenser.

For certain applications, it is useful to have text printed on the separable straps, for instance to provide label information such as product codes, bar codes, identification of bundled elements for telecommunication, instructions or warnings. Such information may be pre-printed on the hook or loop portion of the straps.

Advantageously, a lightweight, non-woven loop material is produced. It comprises a non-woven web of entangled fibers of substantial tenacity, the fibers forming a sheet-form body and hook-engageable free-standing loops extending from the web body. The web is stretched before bonding to produce spaced apart loop clusters extending from a very thin web of taut fibers, and binder can be added to stabilize the product in its stretched condition. The backside of the loop product is then combined with the back side of a layer that carries hook fasteners; this combining step can be effective to bind the knots to hold the fabric in its stretched condition, to serve the function of the binder just mentioned.

This hook-engageable material is then used for forming a composite product having a large multiplicity of hook-form fastener projections extending from the opposite surface. For this purpose a cooled, rotating mold roller is provided having inwardly extending, fixed, projection-forming cavities defined in its periphery, to the exterior of the forming roller, molten plastic material is applied for filling the cavities and forming a base in the manner that incorporates the hook engageable material on the side of the base opposite from the side in which the projections are formed, the fastener material is withdrawn from the forming roller in a step that includes withdrawing the projections from the cavities, and either in line or as a separate batch process, parting lines are formed at spaced intervals extending transversely of the strip material.

Advantageously the non-woven material is laminated in situ with the hook material using a layer of resin that forms the base of the hook material to bond directly to the non-woven.

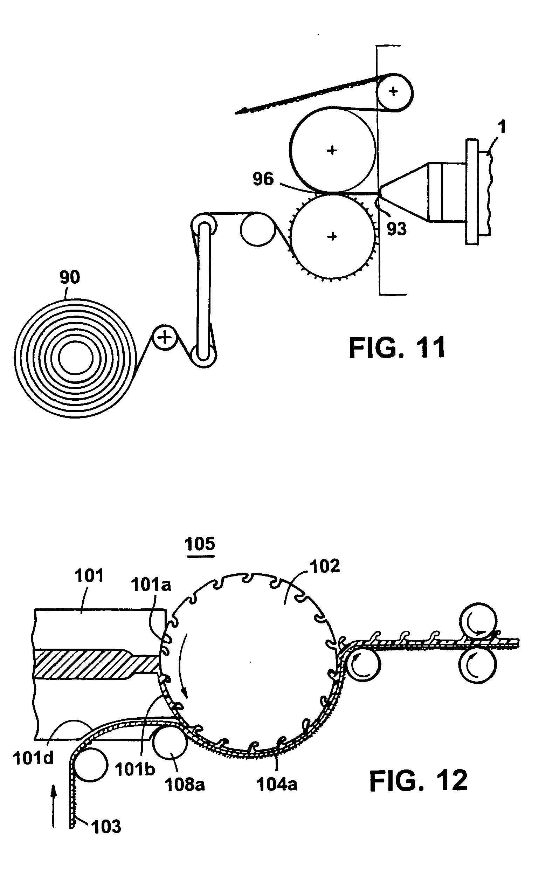

In the case of using the specific example FIG. 11 of this application, a pre-formed sheet material 90 is forced into the nip 96'at the same time as molten plastic 93 is forced into the nip 96 to create strip fastener tape, the sheet material bonding intimately with the fastener to become an integral part of the structure of the strip fastener.

At the front end surface of the upper half of the extrusion nozzle 101 shown in FIG. 12 of this application, there is an upper arced surface 101a, the curve of which mirrors that of the die wheel 102, while at the front end surface of the lower half of the nozzle 101, there is a further lower arced surface 101b, the curve of which also mirrors that of the die wheel.

A guide channel 101d through which the non-woven material is introduced is formed in the lower half of the extrusion nozzle 101. A rear pressure roller 108a applies pressure between the sheet of molten resin extruded from the extrusion nozzle, to join the non-woven material 103 to be attached to the resin.

Thus in operation the molten resin extruded from the extrusion nozzle 101 is forced into the gap between the die wheel 102 and the lower arced surface 101b, and fills up the hook molding cavities 105 along with a base layer 104a of fixed thickness and width. At the same time as this molding process is taking place, the backing material 103 is guided up through the backing material guide channel 101d in the extrusion nozzle 101 and is pressed against the surface of the molten base layer 104a by the rear pressure roller 108a to firmly join them together, so that the bases of the loops in the backing material are firmly held by the resin so that the loops will hold their shape well and will be very durable.

Various preferred embodiments of the invention have been presented. However, the illustrations are not exhaustive; it should be readily apparent to one skilled in the art that other configurations of the dispenser and the strap material are possible, including use of alternative strap material configurations and arrangements within the dispenser.

## Claims

1. A fastener strap dispenser (10, 200, 300) comprising:
a housing (11, 202) defining a receptacle (12, 204) and an outlet (32, 227); a length of fastener tape (14, 206) disposed within the receptacle and arranged to be dispensed through the outlet, the tape comprising a strip-form base (82) having two broad, opposite surfaces (81, 83), an array of fastener elements (80, 84) extending from one of the surfaces,
the strip-form base (82) of the fastener tape (14, 206) defining tear regions (50) of reduced tear strength formed at spaced intervals along the length defining individual fastener straps therebetween with each fastener strap defining an associated, engageable formation (52, 52'); and
the fastener strap dispenser (10; 200; 300) comprising a detent (40, 246) constructed and positioned to engage the engageable formation (52, 52') of a trailing one of said straps as a leading one of said straps is pulled through the outlet (32, 227), the detent acting to resist movement of the trailing strap so that the leading strap parts from the trailing strap at a tear region (50) when a sufficient parting tension is applied to the leading strap.

2. The fastener strap dispenser (10, 200, 300) of claim 1 further comprising a retarder (17, 26, 224) coupled to the housing (11, 202) and arranged to provide drag against motion of the fastener tape (14, 206) as the leading strap is withdrawn through the outlet (32, 227).

3. The fastener strap dispenser (10, 200, 300) of claim 2 in which the retarder (17, 26, 224) comprises a resilient member (17, 26, 224) that bears against the fastener tape material (14, 206) as the leading strap is withdrawn.

4. The fastener strap dispenser (10, 200, 300) of claim 3 in which said resilient member (17, 26, 224) is a leaf spring (17, 224) or a spring-loaded roller (26).

5. The fastener strap dispenser (10, 200, 300) of any of the preceding claims wherein the detent (40, 246) comprises a fixed protrusion (40, 246) over which the fastener tape (14, 206) slides and the engageable formations (52, 52') comprise slits defined by the fastener tape, the protrusion arranged to engage the slit of the trailing strap as the leading strap is pulled from the outlet (32, 227).

6. The fastener strap dispenser (10, 200, 300) of claims 3 and 5 wherein the resilient member (17, 26, 224) is positioned to bias the fastener tape (14, 206) toward the fixed protrusion (40, 246) as the leading strap is withdrawn from the outlet (32, 227) to aid engagement of the engageable formation (52, 52') of the trailing strap by the fixed protrusion.

7. The fastener strap dispenser (10, 200, 300) of claims 3 and 5 wherein the resilient member (17, 26, 224) is positioned to bias the fastener tape (14, 206) away from the fixed protrusion (40, 246) as the leading strap is withdrawn from the outlet (32, 227) to aid disengagement of the engageable formation (52, 52') of the trailing strap from the fixed protrusion.

8. The fastener strap dispenser (10, 200, 300) of any of claims 5 to 7 wherein the housing (11, 202) defines a guide channel (34, 234) to position the engageable formations (52, 52') for engagement with the fixed protrusion (40, 246) as the fastener tape (14, 206) is dispensed.

9. The fastener strap dispenser (10, 200, 300) of any of the preceding claims wherein the dispenser is proportioned to be hand-held by a user, preferably having a total weight of less than about 0.5 kg (one pound), and preferably having a maximum linear dimension of about 127 mm (5 inches).

10. The fastener strap dispenser (10, 200, 300) of any of the preceding claims wherein the housing has a curved outer surface (216) to receive an outer surface of an article to be wrapped (213).

11. A fastener strap dispenser (10, 200, 300) of any of claims 2 to 10, wherein the retarder (17, 26, 224 is manipulable by a user to provide increased resistance to a trailing one of said fastener straps to facilitate separation of a leading one of said fastener straps from the trailing strap at an associated tear region when a sufficient parting tension is applied to the leading strap.

12. The fastener strap dispenser (300) according to any of the preceding claims further comprising:
a severing device (323) at the outlet of the housing, the severing device having a sharp edge (327) for cutting through the base (82) of the tape when forced against one of the broad surfaces (81, 83) of the tape.

13. The fastener strap dispenser (300) of claim 12 wherein said severing device (323) is an exposed edge of said retarder (324).

14. A length of fastener tape (14, 206) to be used with a fastener strap dispenser (10; 200; 300) according to any of the precending claims, comprising:
an elongated strip-form base (82) having a uniform width, two broad, opposite surfaces (81, 83) and tear regions (50) of reduced tear strength formed at spaced intervals along its length, the tear regions defining individual fastener straps therebetween, the strip-form base defining an engageable formation (52, 52') for engaging the strip-form base to the detent (40, 246) of the dispenser (10, 200, 300) to tear the fastener tape along an associated tear region; and
an array of fastener elements (80, 84) extending from at least one of the broad surfaces of the base.

15. The length of fastener tape (14, 206) of claim 14 wherein the engageable formations (52, 52') comprise slits (52, 52') extending through the base.

16. The length of fastener tape (14, 206) of claim 15 wherein the slits (52, 52') are u-shaped (52').

17. The length of fastener tape (14, 206) of any of claims 14 to 16 wherein the base (82) and array of fastening elements (80, 84) are a single, unitarily molded structure.

18. The length of fastener tape (14, 206) of any of claims 14 to 17 wherein each of the broad surfaces (81, 83) of the tape have fastening elements (80, 84) extending therefrom, the fastening elements of one of the broad surfaces being adapted to engage the fastening elements of the other broad surface.

19. The length of fastener tape (14, 206) of claim 18 wherein the fastening elements (80, 84) of one broad surface are loops (84) and the fastening elements of the other broad surface are loop-engaging fastener elements (80).

20. The length of fastener tape (14, 206) of any of claims 14 to 19 in which the engageable formations (52, 52') of the fastener straps are located at the tear regions (50).

21. The length of fastener tape (14, 206) of any of claims 14 to 19 in which the engageable formations (52, 52') of the fastener straps are longitudinally spaced from the tear regions (50).

22. The length of fastener tape (14, 206) of any of claims 14 to 21 wherein the fastener elements (80, 84) comprise hooks (80).

23. The length of fastener tape (14, 206) of any of claims 14 to 22 wherein the fastener elements (80, 84) comprise fibers (84) adapted to be engaged by hooks for hook and loop fastening.

24. The length of fastener tape (14, 206) of any of claims 14 to 23 wherein the fastener tape (14, 206) has loops (84) on one broad surface (81 or 83) thereof, and loop-engageable fastener elements (80) on an opposite broad surface (81 or 83) thereof.

25. A method of wrapping an article (213) comprising:
providing the fastener strap dispenser (10, 200, 300) of any of claims 1 to 13;
pulling a free end (221) of a leading fastener strap from the dispenser (10, 200, 300) until the engageable formation (52, 52') of a trailing strap is engaged by the detent (40, 246);
wrapping the leading fastener about the article (213); and
applying sufficient tension to separate the leading strap from the trailing strap at an exposed tear region (50).

## Patentansprüche

1. Befestigungsbandspender (10, 200, 300) umfassend:
ein Gehäuse (11, 202), das einen Behälter (12, 204) und einen Auslaß (32, 227) definiert; wobei eine Befestigungsbandlänge (14, 206) in dem Behälter vorgesehen und angeordnet ist, um durch den Auslaß ausgegeben zu werden, wobei das Band eine streifenförmige Basis (82), die zwei breite gegenüberliegende Oberflächen (81, 83) aufweist, und ein Feld bzw. Array von Festlegungs- bzw. Befestigungselementen (80, 84) umfaßt, die sich von einer der Oberflächen erstrecken,
wobei die streifenförmige Basis (82) des Befestigungsbands (14, 206) Reißbereiche (50) einer reduzierten Reißfestigkeit definiert, die an beabstandeten Intervallen entlang der Länge ausgebildet sind, die individuelle Befestigungsstreifen dazwischen definiert, wobei jeder Befestigungsstreifen eine assoziierte bzw, zugehörige, ergreifbare Formation bzw. Ausbildung (52, 52') definiert; und
wobei der Befestigungsbandspender (10; 200; 300) eine Feststellvorrichtung (40, 246) umfaßt, die konstruiert ist und positioniert ist, um die ergreifbare Formation (52, 52') eines nachlaufenden Streifens zu ergreifen, wenn ein vorderer der Streifen durch den Auslaß (32, 227) gezogen ist, wobei die Feststellvorrichtung wirkt, um einer Bewegung des nachlaufenden Streifens zu widerstehen, so daß sich der vordere Streifen von dem nachlaufenden Streifen an einem Abrißbereich (50) trennt, wenn eine ausreichende Trennspannung auf den vorderen Streifen aufgebracht ist.

2. Befestigungsbandspender (10, 200, 300) nach Anspruch 1, weiterhin umfassend eine Verzögerungseinrichtung (17, 26, 224), die mit dem Gehäuse (11, 202) gekoppelt und angeordnet ist, um einen Widerstand gegenüber einer Bewegung des Befestigungsbands (14, 206) zur Verfügung zu stellen, wenn der vordere Streifen durch den Auslaß (32, 227) herausgezogen ist bzw. wird.

3. Befestigungsbandspender (10, 200, 300) nach Anspruch 2, in welchem die Verzögerungseinrichtung (17, 26, 224) ein rückstellfähiges Glied (17, 26, 224) umfaßt, welches gegen das Befestigungsbandmaterial (14, 206) aufliegt, wenn der vordere Streifen herausgezogen ist bzw. wird.

4. Befestigungsbandspender (10, 200, 300) nach Anspruch 3, in welchem das rückstellfähige Glied (17, 26, 224) eine Blattfeder (17, 224) oder eine federbelastete Walze (26) ist.

5. Befestigungsbandspender (10, 200, 300) nach einem der vorhergehenden Ansprüche, wobei die Feststellvorrichtung (40, 246) einen festgelegten Vorsprung (40, 246) aufweist, über welchen das Befestigungsband (14, 206) gleitet, und die in Eingriff bringbaren bzw. ergreifbaren Formationen (52, 52') Schlitze umfassen, die durch das Befestigungsband definiert sind, wobei der Vorsprung zum Ergreifen des Schlitzes des nachlaufenden Streifens angeordnet ist, wenn der vordere Streifen aus dem Auslaß (32, 227) herausgezogen ist.

6. Befestigungsbandspender (10, 200, 300) nach Anspruch 3 und 5, wobei das rückstellfähige Glied (17, 26, 224) positioniert ist, um das Befestigungsband (14, 206) in Richtung zu dem festgelegten Vorsprung (40, 246) vorzuspannen bzw. zu beaufschlagen, wenn der vordere Streifen an dem Auslaß (32, 227) herausgezogen ist, um einen Eingriff der ergreifbaren bzw. in Eingriff bringbaren Formation (52, 52') des nachlaufenden Streifens durch den festgelegten Vorsprung zu unterstützen.

7. Befestigungsbandspender (10, 200, 300) nach Anspruch 3 und 5, wobei das rückstellfähige Glied (17, 26, 224) positioniert ist, um das Befestigungsband (14, 206) weg von dem festgelegten Vorsprung (40, 246) vorzuspannen bzw. zu beaufschlagen, wenn der vordere Streifen aus dem Auslaß (32, 227) herausgezogen ist, um ein Lösen der ergreifbaren Formation (52, 52') des nachlaufenden Streifens von dem festgelegten Vorsprung zu unterstützen.

8. Befestigungsbandspender (10, 200, 300) nach einem der Ansprüche 5 bis 7, wobei das Gehäuse (11, 202) einen Führungskanal (34, 234) definiert, um die eingreifbaren Formationen (52, 52') für einen Eingriff mit dem festgelegten Vorsprung (40, 246) zu positionieren, wenn das Befestigungsband (14, 206) ausgegeben ist bzw. wird.

9. Befestigungsbandspender (10, 200, 300) nach einem der vorhergehenden Ansprüche, wobei der Spender proportioniert ist, um durch einen Benutzer in der Hand gehalten zu sein, wobei er vorzugsweise ein Gesamtgewicht von weniger als etwa 0,5 kg (ein Pfund) aufweist, und vorzugsweise eine maximale lineare Abmessung von etwa 127 mm (5 Zoll) aufweist.

10. Befestigungsbandspender (10, 200, 300) nach einem der vorhergehenden Ansprüche, wobei das Gehäuse eine gekrümmte Außenoberfläche (216) aufweist, um eine Außenoberfläche eines zu umwickelnden Gegenstands (213) aufzunehmen.

11. Befestigungsbandspender (10, 200, 300) nach einem der Ansprüche 2 bis 10, wobei die Verzögerungseinrichtung (17, 26, 224) durch einen Benutzer handhabbar ist, um einen erhöhten Widerstand gegenüber einem nachlaufenden Befestigungsstreifen zur Verfügung zu stellen, um eine Trennung eines vorderen der Befestigungsstreifen von dem nachlaufenden Streifen an einem assoziierten Reißbereich zu erleichtern, wenn eine ausreichende Trennspannung auf den vorderen bzw. voreilenden Streifen aufgebracht ist.

12. Befestigungsbandspender (300) nach einem der vorhergehenden Ansprüche, weiterhin umfassend:
eine Trenn- bzw. Zerteilvorrichtung (323) am Auslaß des Gehäuses, wobei die Zerteilvorrichtung eine scharfe Kante (327) zum Schneiden durch die Basis (82) des Bands aufweist, wenn sie gegen eine der breiten Oberflächen (81, 83) des Bands gezwungen bzw. beaufschlagt ist.

13. Befestigungsbandspender (300) nach Anspruch 12, wobei die Zerteilvorrichtung (323) eine freigelegte Kante der Verzögerungseinrichtung (324) ist.

14. Länge eines Befestigungsbands (14, 206), das mit einem Befestigungsbandspender (10; 200; 300) gemäß einem der vorhergehenden Ansprüche zu verwenden ist, umfassend:
eine längliche streifenförmige Basis (82), die eine gleichmäßige Breite aufweist, zwei breite gegenüberliegende Oberflächen (81, 83) und Reiß- bzw. Abrißbereiche (50) mit reduzierter Reißfestigkeit, die an beabstandeten Intervallen entlang seiner Länge ausgebildet sind, wobei die Abrißbereiche individuelle Befestigungsstreifen dazwischen definieren, wobei die streifenförmige Basis eine ergreifbare Formation bzw. Ausbildung (52, 52') für ein Ergreifen der streifenförmigen Basis an der Feststellvorrichtung (40, 246) des Spenders (10, 200, 300) definiert, um den Befestigungsstreifen entlang eines assoziierten bzw. zugehörigen Abrißbereichs zu zerreißen; und
ein Feld bzw. Array von Befestigungselementen (80, 84), die sich von wenigstens einer der breiten Oberflächen der Basis erstrecken.

15. Befestigungsbandlänge (14, 206) nach Anspruch 14, wobei die ergreifbaren Formationen (52, 52') Schlitze (52, 52') umfassen, die sich durch die Basis erstrecken.

16. Befestigungsbandlänge (14, 206) nach Anspruch 15, wobei die Schlitze (52, 52') u-förmig (52') sind.

17. Befestigungsbandlänge (14, 206) nach einem der Ansprüche 14 bis 16, wobei die Basis (82) und das Feld von Befestigungsstreifen (80, 84) eine einzelne einstückig geformte Struktur aufweisen.

18. Befestigungsbandlänge (14, 206) nach einem der Ansprüche 14 bis 17, wobei jede der breiten Oberflächen (81, 83) des Bands Befestigungselemente (80, 84) aufweist, die sich davon erstrecken, wobei die Festlegungs- bzw. Befestigungselemente von einer der breiten Oberflächen adaptiert ist, um die Befestigungselemente der anderen breiten Oberfläche zu ergreifen.

19. Befestigungsbandlänge (14, 206) nach Anspruch 18, wobei die Befestigungselemente (80, 84) einer breiten Oberfläche Schlaufen (84) sind und die Befestigungselemente der anderen breiten Oberfläche schlaufenergreifende Befestigungselemente (80) sind.

20. Befestigungsbandlänge (14, 206) nach einem der Ansprüche 14 bis 19, in welcher die ergreifbaren Formationen (52, 52') der Befestigungsstreifen an den Abrißbereichen (50) angeordnet sind.

21. Befestigungsbandlänge (14, 206) nach einem der Ansprüche 14 bis 19, in welcher die ergreifbaren Formationen (52, 52') der Befestigungsstreifen in Längsrichtung von den Abrißbereichen (50) beabstandet sind.

22. Befestigungsbandlänge (14, 206) nach einem der Ansprüche 14 bis 21, wobei die Befestigungselemente (80, 84) Haken umfassen (80).

23. Befestigungsbandlänge (14, 206) nach einem der Ansprüche 14 bis 22, wobei die Befestigungselemente (80, 84) Fasern (84) umfassen, die adaptiert sind, um durch Haken für eine Haken- und Schlaufenbefestigung ergriffen zu werden.

24. Befestigungsbandlänge (14, 206) nach einem der Ansprüche 14 bis 23, wobei das Befestigungsband (14, 206) Schlaufen (84) auf einer breiten Oberfläche (81 oder 83) davon und schlaufenergreifende Befestigungselemente (80) auf einer gegenüberliegenden breiten Oberfläche (81 oder 83) davon aufweist.

25. Verfahren zum Verpacken eines Gegenstands bzw. Artikels (213) umfassend:
Bereitstellen eines Befestigungsstreifenspenders (10, 200, 300) nach einem der Ansprüche 1 bis 13;
Ziehen eines freien Endes (221) eines vorderen Befestigungsstreifens aus dem Spender (10, 200, 300), bis die ergreifbare Formation (52, 52') eines nachlaufenden Streifens durch die Feststellvorrichtung (40, 246) ergriffen wird;
Umwickeln des vorderen Befestigungselements um den Gegenstand (213); und
Anwenden eines ausreichenden Zugs, um den vorderen Streifen von dem nachlaufenden Streifen an einem freigelegten Abrißbereich (50) zu trennen.

## Revendications

1. Distributeur de bande de fixation (10, 200, 300) comprenant :
- un boîtier (11, 202) qui définit un récipient (12, 204) et un orifice de sortie (32, 227) ;
- une longueur de bande de fixation (14, 206) disposée à l'intérieur du récipient et mise au point pour être distribuée à travers l'orifice de sortie, la bande comprenant une base en forme de bandelette (82) ayant deux grandes surfaces opposées (81, 83), une rangée d'éléments de fixation (80, 84) s'étendant depuis une des surfaces ;
- la base en forme de bandelette (82) de la bande de fixation (14, 206) définissant des régions à rupture (50) présentant une résistance diminuée à la rupture, formées à intervalles espacés sur la longueur qui définit des bandes de fixation individuelles entre celles-ci, chaque bande de fixation définissant une conformation associée et pouvant être engagée (52, 52') ; et
- le distributeur de bande de fixation (10 ; 200 ; 300) comprenant un positionneur (40, 246) construit et positionné pour engager la conformation pouvant être engagée (52, 52') d'une bande traînante parmi lesdites bandes, alors qu'une bande en avancement parmi lesdites bandes est tirée à travers l'orifice de sortie (32, 227), le positionneur agissant pour résister au mouvement de la bande traînante de sorte que la bande en avancement se sépare de la bande traînante sur une région à rupture (50) lorsqu'une tension de séparation suffisante est appliquée sur la bande en avancement.

2. Distributeur de bande de fixation (10, 200, 300) selon la revendication 1, comprenant en outre un ralentisseur (17, 26, 224) couplé au boîtier (11, 202) et mis au point pour fournir une traînée contre le mouvement de la bande de fixation (14, 206) alors que la bande en avancement est détachée par soulèvement à travers l'orifice de sortie (32, 227).

3. Distributeur de bande de fixation (10, 200, 300) selon la revendication 2, dans lequel le ralentisseur (17, 26, 224) comprend un élément résilient (17, 26, 224) qui s'appuie contre le matériau de bande de fixation (14, 206) alors que la bande en avancement est détachée par soulèvement.

4. Distributeur de bande de fixation (10, 200, 300) selon la revendication 3, dans lequel ledit élément résilient (17, 26, 224) est un ressort à lame (17, 224) ou un rouleau chargé par ressort (26).

5. Distributeur de bande de fixation (10, 200, 300) selon l'une quelconque des revendications précédentes, dans lequel le positionneur (40, 246) comprend une saillie fixée (40, 246) sur laquelle glisse la bande de fixation (14, 206), et dans lequel les conformations pouvant être engagées (52, 52') comprennent des fentes définies par la bande de fixation, la saillie étant ménagée pour engager la fente de la bande traînante alors que la bande en avancement est tirée à travers l'orifice de sortie (32, 227).

6. Distributeur de bande de fixation (10, 200, 300) selon les revendications 3 et 5, dans lequel l'élément résilient (17, 26, 224) est positionné pour incliner la bande de fixation (14, 206) vers la saillie fixée (40, 246) alors que la bande en avancement est détachée par soulèvement de l'orifice de sortie (32, 227) pour contribuer à l'engagement de la conformation pouvant être engagée (52, 52') de la bande traînante par la saillie fixée.

7. Distributeur de bande de fixation (10, 200, 300) selon les revendications 3 et 5, dans lequel l'élément résilient (17, 26, 224) est positionné pour incliner la bande de fixation (14, 206) à l'écart de la saillie fixée (40, 246) alors que la bande en avancement est détachée par soulèvement de l'orifice de sortie (32, 227) pour contribuer au désengagement de la conformation pouvant être engagée (52, 52') de la bande traînante depuis la saillie fixée.

8. Distributeur de bande de fixation (10, 200, 300) selon l'une quelconque des revendications 5 à 7, dans lequel le boîtier (11, 202) définit un canal de guidage (34, 234) pour positionner les conformations pouvant être engagées (52, 52') pour l'engagement avec la saillie fixée (40, 246), alors que la bande de fixation (14, 206) est distribuée.

9. Distributeur de bande de fixation (10, 200, 300) selon l'une quelconque des revendications précédentes, dans lequel le distributeur est proportionné pour être tenu à la main par un utilisateur, en ayant de préférence un poids total de moins de 0,5 kg (une livre), et en ayant de préférence une dimension linéaire maximale d'environ 127 mm (5 pouces).

10. Distributeur de bande de fixation (10, 200, 300) selon l'une quelconque des revendications précédentes, dans lequel le boîtier a une surface extérieure courbée (216) pour recevoir une surface extérieure d'un article devant être enroulé (213).

11. Distributeur de bande de fixation (10, 200, 300) selon l'une quelconque des revendications 2 à 10, dans lequel le ralentisseur (17, 26, 224) peut être manipulé par un utilisateur pour fournir une résistance augmentée à une bande traînante parmi lesdites bandes de fixation afin de faciliter la séparation d'une bande en avancement parmi lesdites bandes de fixation, depuis la bande traînante sur une région à rupture associée lorsqu'une tension de séparation suffisante est appliquée sur la bande en avancement.

12. Distributeur de bande de fixation (300) selon l'une quelconque des revendications précédentes, comprenant en outre un dispositif de rupture (323) sur l'orifice de sortie du boîtier, le dispositif de rupture ayant un bord acéré (327) pour sectionner la base (82) de la bande lorsqu'une force est appliquée contre l'une des grandes surfaces (81, 83) de la bande.

13. Distributeur de bande de fixation (300) selon la revendication 12, dans lequel ledit dispositif de rupture (323) est un bord dégagé dudit ralentisseur (224).

14. Longueur de bande de fixation (14, 206) devant être utilisée avec un distributeur de bande de fixation (10 ; 200 ; 300) selon l'une quelconque des revendications précédentes, comprenant :
- une base allongée en forme de bandelette (82) ayant une largeur uniforme, deux grandes surfaces opposées (81, 83) et des régions à rupture (50) présentant une résistance réduite à la rupture, formées à intervalles espacés sur sa longueur, les régions à rupture définissant des bandes de fixation individuelles entre elles, la base en forme de bandelette définissant une conformation pouvant être engagée (52, 52') pour l'engagement de la base en forme de bandelette vers le positionneur (40, 246) du distributeur (10, 200, 300) pour rompre la bande de fixation le long d'une région de rupture associée ; et
- une rangée d'éléments de fixation (80, 84) s'étendant depuis au moins une des grandes surfaces de la base.

15. Longueur de bande de fixation (14, 206) selon la revendication 14, dans laquelle les conformations pouvant être engagées (52, 52') comprennent des fentes (52, 52') s'étendant à travers la base.

16. Longueur de bande de fixation (14, 206) selon la revendication 15, dans laquelle les fentes (52, 52') sont en forme de U (52').

17. Longueur de bande de fixation (14, 206) selon l'une quelconque des revendications 14 à 16, dans laquelle la base (82) et une rangée d'éléments de fixation (80, 84) sont une structure unique, à moulage unitaire.

18. Longueur de bande de fixation (14, 206) selon l'une quelconque des revendications 14 à 17, dans laquelle chacune des grandes surfaces (81, 83) de la bande a des éléments de fixation (80, 84) s'étendant depuis celles-ci, les éléments de fixation de la une des grandes surfaces étant adaptés pour engager les éléments de fixation de l'autre grande surface.

19. Longueur de bande de fixation (14, 206) selon la revendication 18, dans laquelle les éléments de fixation (80, 84) d'une grande surface sont des boucles (84) et les éléments de fixation de l'autre grande surface sont des éléments de fixation (80) s'engageant dans les boucles.

20. Longueur de bande de fixation (14, 206) selon l'une quelconque des revendications 14 à 19, dans laquelle les conformations pouvant être engagées (52, 52') des bandes de fixation sont placées sur les régions à rupture (50).

21. Longueur de bande de fixation (14, 206) selon l'une quelconque des revendications 14 à 19, dans laquelle les conformations pouvant être engagées (52, 52') des bandes de fixation sont espacées des régions à rupture (50) en direction longitudinale.

22. Longueur de bande de fixation (14, 206) selon l'une quelconque des revendications 14 à 21, dans laquelle les éléments de fixation (80, 84) comprennent des crochets (80).

23. Longueur de bande de fixation (14, 206) selon l'une quelconque des revendications 14 à 22, dans laquelle les éléments de fixation (80, 84) comprennent des fibres (84) adaptées pour être engagées par des crochets pour une fixation par crochet et boucle.

24. Longueur de bande de fixation (14, 206) selon l'une quelconque des revendications 14 à 23, dans laquelle la bande de fixation (14, 206) a des boucles (84) sur une grande surface (81 ou 83) de celle-ci, et a des éléments de fixation (80) pouvant être engagés dans les boucles sur la grande surface opposée (81 ou 83).

25. Méthode d'enroulement d'un article (213) comprenant :
- la fourniture d'un distributeur de bande de fixation (10, 200, 300) selon l'une quelconque des revendications 1 à 13 ;
- le tirage d'une extrémité libre (221) d'une bande de fixation en avancement depuis le distributeur (10, 200, 300) jusqu'à ce que la conformation pouvant être engagée (52, 52') d'une bande traînante soit engagée par le positionneur (40, 246);
- l'enroulement de la bande en avancement autour de l'article (213) ; et
- l'application d'une tension suffisante pour séparer la bande en avancement de la bande traînante sur une région à rupture dégagée (50).
